# EUROPEAN PATENT APPLICATION

(11) **EP 4 502 156 A1**
(43) Date of publication of application: **05.02.2025**
(21) Application number: 23780462.0
(22) Date of filing: 28.03.2023
(51) Int. Cl.: C12N 15/09, A01K 67/033, C07K 14/435, C12N 15/12, D01F 4/02

(54) **GENOME-MODIFIED SILKWORM PRODUCING CHIMERIC SILK YARNS**

(30) Priority: 30.03.2022 JP 2022056854
(71) Applicant: NATIONAL AGRICULTURE AND FOOD RESEARCH ORGANIZATION, Tsukuba-shi, Ibaraki 305-8517 (JP); KOWA COMPANY, LTD., Naka-ku Nagoya-shi Aichi 460-8625 (JP)
(72) Inventor: YAMADA, Nobuto, Tsukuba-shi, Ibaraki 305-8634 (JP); TAKASU, Yoko, Tsukuba-shi, Ibaraki 305-8634 (JP); KOJIMA, Katsura, Tsukuba-shi, Ibaraki 305-8634 (JP); YOSHIOKA, Taiyo, Tsukuba-shi, Ibaraki 305-8634 (JP); TSUBOTA, Takuya, Tsukuba-shi, Ibaraki 305-8634 (JP); UCHINO, Keiro, Tsukuba-shi, Ibaraki 305-8634 (JP); SEZUTSU, Hideki, Tsukuba-shi, Ibaraki 305-8634 (JP); KAMEDA, Tsunenori, Tsukuba-shi, Ibaraki 305-8634 (JP); ASANUMA, Akimune, Tsukuba-shi, Ibaraki 305-0856 (JP); MURAKAMI, Takeshi, Tsukuba-shi, Ibaraki 305-0856 (JP); DOI, Takeshi, Tsukuba-shi, Ibaraki 305-0856 (JP)
(74) Representative: Mewburn Ellis LLP
(86) International application number: PCT/JP2023/012392
(87) International publication number: WO 2023/190453

(57) **Abstract**

A silkworm that can produce a chimeric silk thread comprising a silkworm silk thread is created, the physical properties of which chimeric silk thread are approximate to those of the bagworm silk thread. From the silkworm, a chimeric silk thread of interest is obtained in fibrous form. Provided is a genome-modified silkworm in which a gene encoding a modified bagworm fibroin protein is inserted, wherein the gene is obtained by fusing a gene fragment encoding a bagworm-derived fibroin protein and a gene fragment encoding a silkworm-derived fibroin protein.

## Description

### Technical Field

The present invention relates to: a method of producing a genome-modified silkworm that produces a chimeric silk thread; a kit for producing the genome-modified silkworm; the genome-modified silkworm; and a chimeric silk thread obtained from the genome-modified silkworm.

### Background Art

A bagworm collectively refers to a moth larva belonging to the family Psychidae in the order Lepidoptera. A bagworm silk thread spun by this bagworm has higher physical properties than a silk thread spun by a silkworm, and hence, has been attracting attention as a useful novel animal natural fiber in recent years (Patent Literature 1 and Non-Patent Literature 1).

There are some problems to be solved so that a bagworm silk thread can be put to practical use as a fibrous material. One of the problems is a problem with mass production. To obtain bagworm silk threads from bagworms in mass production, a technology of rearing a large number of bagworms and a technology of collecting silk threads from bagworms efficiently are essential. However, the bagworm silk thread industry has just begun. Hence, many of such technologies are in a developing stage, and such mass production will still take time to actualize.

Examples of a method of mass-producing bagworm silk threads comprise not only such a method in which silk threads are directly obtained from bagworms as above-described but also a method in which a fibroin protein (herein often referred to as a "Fib protein"), which is a fibrous component of a bagworm silk thread, is produced in a host using a genetic recombination technology or the like. Introducing a cloned fibroin gene (herein often referred to as a cloned "Fib gene") into a host to allow the gene to be expressed makes it possible to mass-produce a recombinant bagworm Fib protein in the host cell. However, this technology has a problem in that the full-length genome sequence of a fibroin H chain protein (herein often referred to as a "Fib H protein"), which is a main component of a bagworm silk thread, has not been determined sufficiently. This is because the Fib H protein has an amino acid sequence consisting of many repeated glycine residues and alanine residues, thus making it extremely difficult to determine the base sequence using a usual cloning technology. Additionally, a bagworm Fib protein (herein often referred to as a "bFib protein") expressed in a host is liquid, and thus, has to be processed into fibrous form to be utilized as fiber. However, this method causes a problem of entailing labor and production costs. Furthermore, it is difficult to fiberize a bFib protein using a conventional spinning technology, causing another problem of necessitating the development of a new spinning technology.

On the other hand, silkworms have been reared over 5000 years or more as a domestic insect for collecting a silk thread, and technologies of mass-rearing and mass-thread-production and mass-production facilities have been established. Furthermore, it has been possible in recent years that various genetically modified silk threads are produced from genetically modified silkworms created using a genetic recombination technology.

In view of this, the present inventors have succeeded with an early solution to the problems in the mass-production of bagworm silk threads, the solution comprising: constructing a modified fibroin gene on the basis of information on a partial base sequence of a gene encoding a Fib H protein of a bagworm (herein often referred to as a "bFib H protein"); creating a genetically modified silkworm having the gene introduced thereinto; allowing the silkworm to spin; and obtaining a chimeric silk thread as a silkworm silk thread provided with the physical properties of a bagworm silk thread (Patent Literature 2). Although the physical properties of the chimeric silk thread obtained have part of the characteristics of a bagworm silk thread, there remains a problem from the viewpoint of obtaining a silk thread the physical properties of which are close to those of a bagworm silk thread.

### Citation List

### Patent Literature

Patent Literature 1: JP2018-197415A
Patent Literature 2: WO2018/074403

### Non-Patent Literature

Non-Patent Literature 1: Shigeyosi Ohsaki, 2002, Sen'i Gakkaishi (Sen'i To Kogyo), 58: 74-78

### Summary of Invention

### Technical Problem

A problem to be addressed by the present invention is to create a silkworm, and to obtain a chimeric silk thread of interest in fibrous form from the silkworm, wherein the silkworm can produce the chimeric silk thread that is a silkworm silk thread but has a high ratio of content of bagworm silk thread, thus having physical properties approximate to the physical properties of the bagworm silk thread.

### Solution to Problem

According to Patent Literature 2, a genetically modified silkworm is created by inserting a gene of interest into the genome of a silkworm by a method using a transposon. However, the efficiency of expression of the exogenous gene inserted using a transposon is no more than several percent of the efficiency of expression of an endogenous gene. The present inventors infer that this is the inhibitory cause for providing the chimeric silk thread with the physical properties of a bagworm silk thread. In view of this, the present inventors have attempted to knock-in a modified fibroin gene of interest to the genome of a silkworm by virtue of a genome-editing technology in which a TAL-PITCh method is used instead of a transposon method. As a result, the present inventors have succeeded in creating a genome-modified silkworm having a bagworm modified-fibroin gene inserted therein. The genome-modified silkworm obtained not only has successfully produced a silkworm-bagworm chimeric fibroin protein (herein often referred to as a "chimeric Fib protein") in a silk gland of the silkworm, but also has successfully spun to produce a chimeric Fib protein in silk thread form. The present invention is based on these results of development, and will provide the following items.

(1) A kit for producing a genome-modified silkworm that produces a chimeric fibroin protein comprising a silkworm-derived fibroin protein and a fibroin protein derived from one or a plurality of species of other silk-spinning insects, the production kit comprising a Left-TALEN expression vector, a Right-TALEN expression vector, and a donor vector; the Left-TALEN expression vector comprising a Left-TALEN coding region, wherein the Left-TALEN coding region encodes, in the fibroin gene of the silkworm, a Left-TALE domain and a nuclease domain downstream of the Left-TALE domain, wherein the Left-TALE domain recognizes and binds to the base sequence of the 5'-side TALE binding region located in the 5'-side vicinity of the insertion site of the fibroin gene of another silk-spinning insect; the Right-TALEN expression vector comprising a Right-TALEN coding region, wherein the Right-TALEN coding region encodes, in the fibroin gene of the silkworm, a Right-TALE domain and a nuclease domain downstream of the Right-TALE domain, wherein the Right-TALE domain recognizes and binds to the base sequence of the 3'-side TALE binding region located in the 3'-side vicinity of the insertion site of the fibroin gene of the another silk-spinning insect; and the donor vector comprising a 5'-side TALE corresponding region, a 3'-side TALE corresponding region, a 5'-side homologous region, a 3'-side homologous region, and the fibroin gene derived from the another silk-spinning insect.
(2) The production kit according to (1), wherein the donor vector comprises the 5'-side TALE corresponding region, the 3'-side homologous region, the 5'-side homologous region, and the 3'-side TALE corresponding region located in this order from the 5' side.
(3) The production kit according to (1) or (2), wherein the fibroin protein derived from the another silk-spinning insect is a fibroin H chain protein.
(4) The production kit according to any one of (1) to (3), wherein the another silk-spinning insect is a bagworm.
(5) The production kit according to (4),
   wherein the bagworm fibroin H chain protein is a modified fibroin H chain protein consisting of three or more same and/or different repeat units that are linked,
   wherein the repeat unit consists of a full length of 120 to 178 amino acids, which comprise 30 or more G/A units each consisting of two amino acid residues composed of a glycine residue and an alanine residue, and comprise an alanine cluster comprising 15 to 25 alanine residues at the N-terminal side of the repeat unit.
(6) The production kit according to (5), wherein the alanine cluster consists of the amino acid sequence of SEQ ID NO: 3 or 4.
(7) The production kit according to (5) or (6), wherein the repeat unit is any one or more selected from the amino acid sequences of SEQ ID NOs: 8 to 16.
(8) The production kit according to any one of (4) to (7), wherein the gene encoding the bagworm fibroin H chain protein consists of any one base sequence of SEQ ID NOs: 17 to 25.
(9) A method of producing a genome-modified silkworm that produces, in a silk gland, a chimeric fibroin protein comprising a silkworm-derived fibroin protein and a fibroin protein derived from one or a plurality of species of other silk-spinning insects, the method comprising: a nucleic acid introducing process of introducing a Left-TALEN mRNA or a Left-TALEN expression vector, a Right-TALEN mRNA or a Right-TALEN expression vector, and a donor vector into an egg of a silkworm; a transformant selecting process of selecting, from the silkworm(s) after the nucleic acid introducing process, a transformant(s) comprising a fibroin gene derived from another silk-spinning insect; and a genome-inserted individual selecting process of selecting, from the transformant(s), an individual wherein the fibroin gene of the another silk-spinning insect is inserted at a target position in the genome of the silkworm; wherein the Left-TALEN mRNA comprises a Left-TALE region and a region encoding a nuclease domain, the Left-TALE region encodes, in the fibroin gene of the silkworm, a Left-TALE domain that recognizes and binds to the base sequence of the 5'-side TALE binding region located at the insertion site of the fibroin gene of the another silk-spinning insect, and the Left-TALEN expression vector comprises a promoter, the Left-TALE region located under the expression control by the promoter, and a region encoding a nuclease domain; wherein the Right-TALEN mRNA comprises a Right-TALE region and a region encoding a nuclease domain, the Right-TALE region encodes, in the fibroin gene of the silkworm, a Right-TALE domain that recognizes and binds to the base sequence of the 3'-side TALE binding region located at the insertion site of the fibroin gene of the another silk-spinning insect, and the Right-TALEN expression vector comprises a promoter, the Right-TALE region located under the expression control by the promoter, and a region encoding a nuclease domain; and wherein the donor vector comprises a 5'-side TALE corresponding region, a 3'-side TALE corresponding region, a 5'-side homologous region, a 3'-side homologous region, and the fibroin gene of the another silk-spinning insect.
(10) The production method according to (9), wherein the donor vector comprises the 5'-side TALE corresponding region, the 3'-side homologous region, the 5'-side homologous region, and the 3'-side TALE corresponding region located in this order from the 5' side on the basis of the direction of the sense strand of the fibroin gene of the another silk-spinning insect.
(11) The production method according to (9) or (10), wherein the fibroin protein derived from the another silk-spinning insect is a fibroin H chain protein.
(12) The production method according to any one of (9) to (11), wherein the another silk-spinning insect is a bagworm.
(13) The production method according to (12), wherein the bagworm fibroin H chain protein is a modified fibroin H chain protein consisting of three or more same and/or different repeat units that are linked, wherein the repeat unit consists of a full length of 120 to 178 amino acids, which comprise 30 or more G/A units consisting of two amino acids composed of a glycine residue and an alanine residue, and comprise an alanine cluster comprising 15 to 25 alanine residues at the N-terminal side of the repeat unit.
(14) The production method according to (13), wherein the alanine cluster consists of the amino acid sequence of SEQ ID NO: 3 or 4.
(15) The production method according to (13) or (14), wherein the repeat unit is any one or more selected from the amino acid sequences of SEQ ID NOs: 8 to 16.
(16) The production method according to any one of (12) to (15), wherein the gene encoding the bagworm fibroin H chain protein consists of any one base sequence of SEQ ID NOs: 17 to 25.
(17) A genome-modified silkworm comprising a bagworm-derived fibroin H chain gene in the genome of the silkworm, wherein a fibroin H chain protein according to the gene is produced in a silk gland.
(18) The genome-modified silkworm according to (17), wherein the bagworm-derived fibroin H chain protein is a chimeric fibroin protein comprising a silkworm-derived fibroin protein.
(19) A chimeric silk thread comprising a chimeric fibroin protein wherein a modified bagworm fibroin H chain protein is linked at an arbitrary position with a silkworm fibroin H chain protein or L chain protein, wherein the modified bagworm fibroin H chain protein comprises three or more same and/or different repeat units that are linked, wherein the repeat unit consists of a full length of 120 to 178 amino acids, which comprise 30 or more G/A units consisting of two amino acids composed of a glycine residue and an alanine residue, and comprise an alanine cluster comprising 15 to 25 alanine residues at the N-terminal side of the repeat unit.
(20) The chimeric silk thread according to (19), wherein the alanine cluster consists of the amino acid sequence of SEQ ID NO: 3 or 4.
(21) The chimeric silk thread according to (19) or (20), wherein the repeat unit is any one or more selected from the amino acid sequences of SEQ ID NOs: 8 to 16.

The present specification encompasses the contents disclosed in the specification and/or drawings of Japanese Patent Application No. 2022-056854, on which the priority of the present application is based.

### Brief Description of Drawings

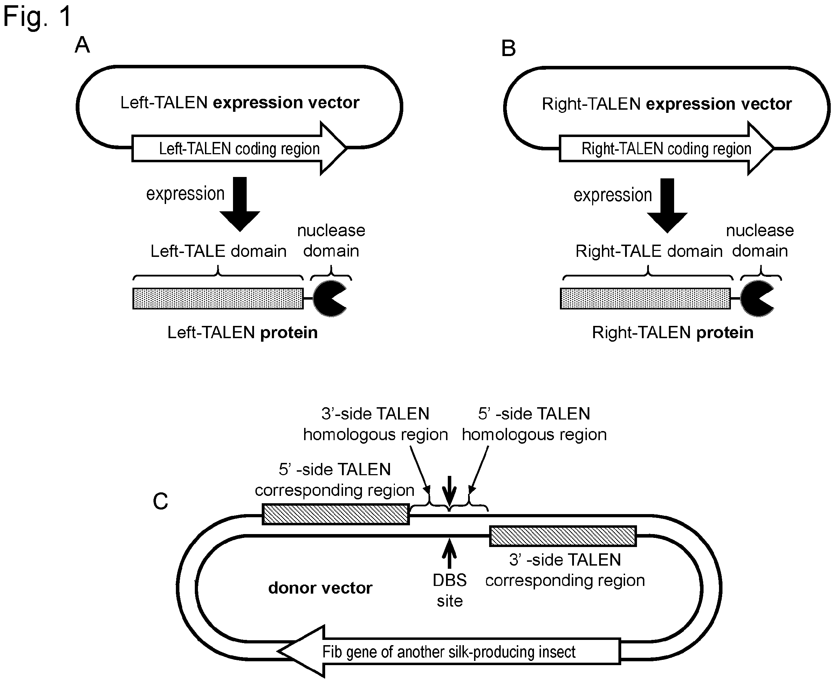

[Figure 1] Figure 1 is a conceptual diagram showing a Left-TALEN expression vector (A), a Right-TALEN expression vector (B), and a donor vector (C) that constitute a kit for producing a genome-modified silkworm according to the present invention.

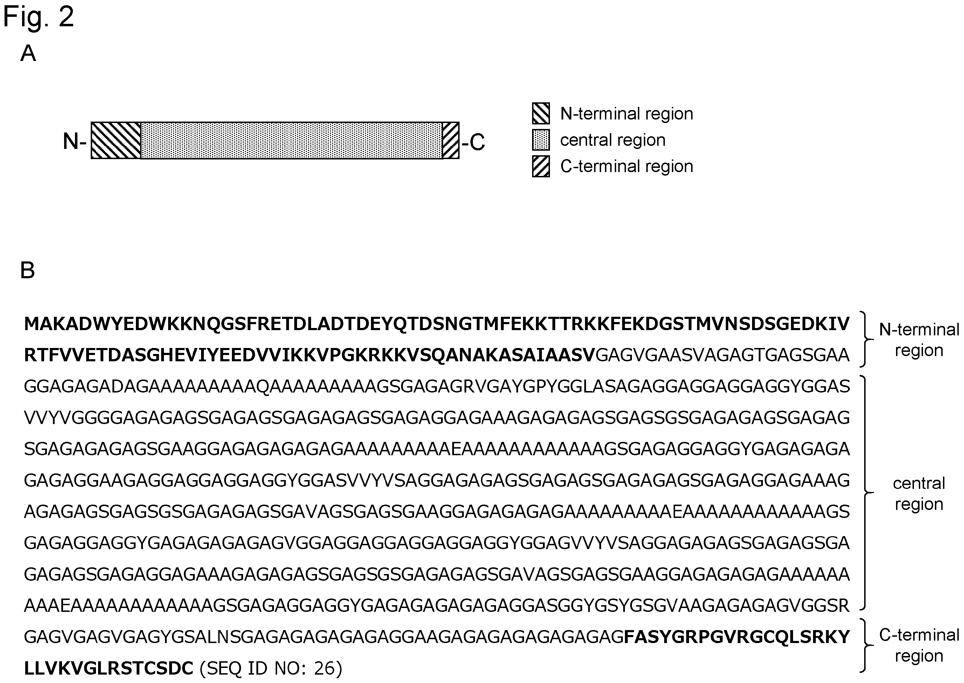

[Figure 2] Figure 2 shows: a schematic diagram (A) illustrating the constitution of a modified bagworm fibroin protein according to WO 2018/07 4403, on which protein the construction of a chimeric protein according to the present invention was based in Example 1; and the amino acid sequence (B) of the protein.

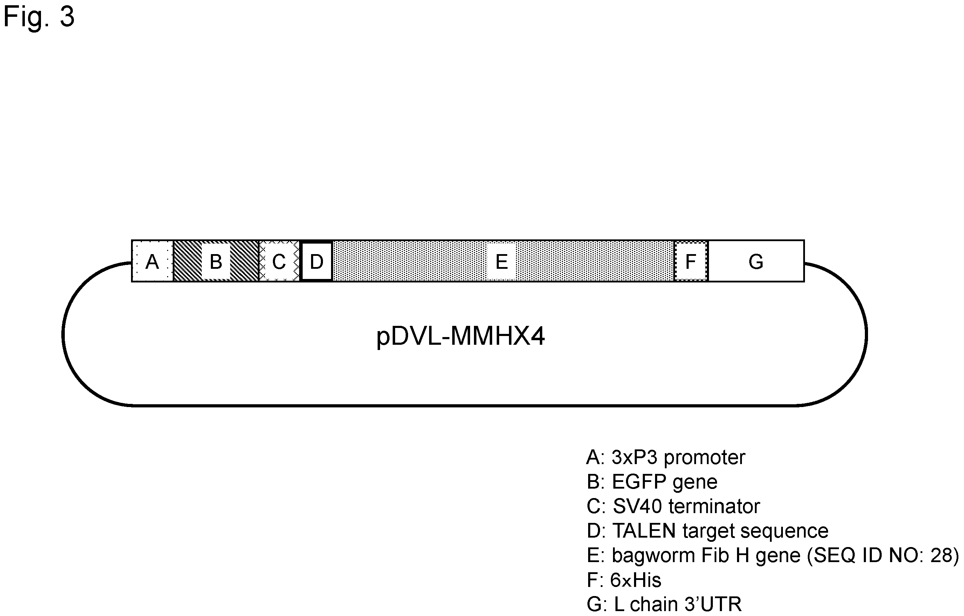

[Figure 3] Figure 3 is a schematic diagram of the donor vector pDVL-MMHX4 according to the present invention, wherein the donor vector was constructed in Example 1.

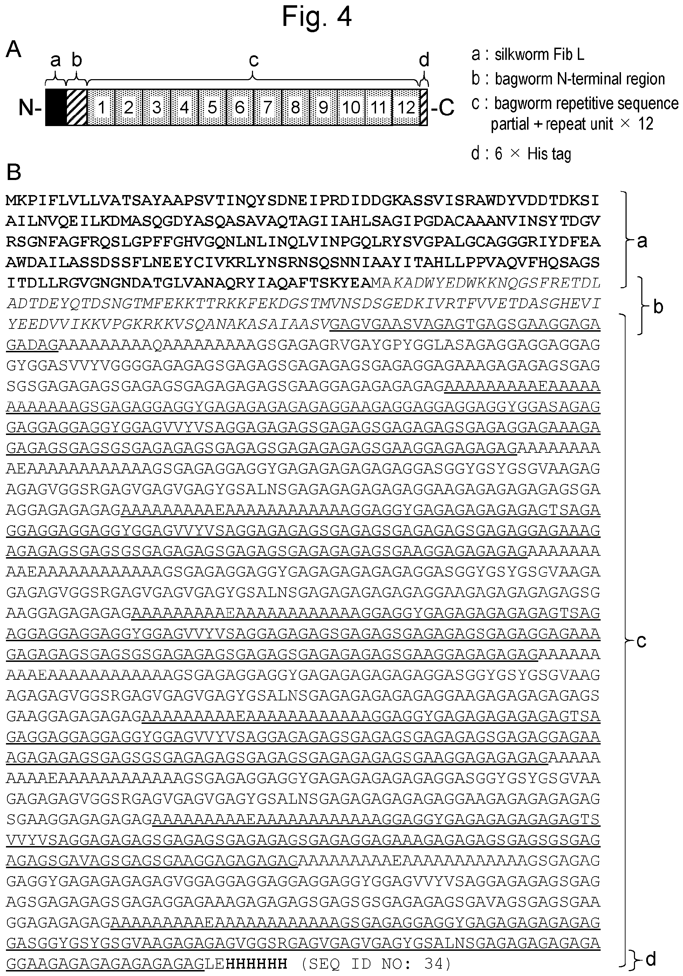

[Figure 4] Figure 4 shows: a schematic diagram (A) illustrating the constitution of a chimeric protein produced by a genome-modified silkworm according to the present invention, wherein the genome-modified silkworm was produced in Example 2; and the amino acid sequence (B, SEQ ID NO: 34) of the chimeric protein. This chimeric protein consists of: the N-terminal region (b) of a modified bagworm fibroin protein, the N-terminal region fused with the C-terminal of a silkworm L chain fibroin (a); repetitive sequences (c: partially repeat units + a repeat unit × 12) of the same fibroin protein; and 6 × a histidine tag (d).

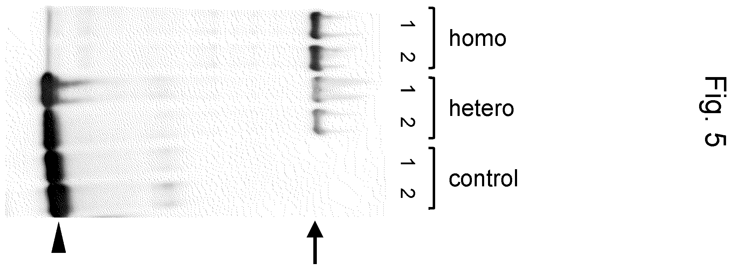

[Figure 5] Figure 5 is a view showing Western blotting performed in Example 2 on the cocoon-shell protein derived from a chimeric silk thread. In the view, the arrow denotes the position of the chimeric L chain produced in Example 1. Additionally, in the hetero-individual and the control individual, bands (arrowhead) were observed at the position of the wild-type silkworm L chain.

### Description of Embodiments

### 1. Genome-Modified Silkworm Production Kit

### 1-1. Overview

A first aspect of the present invention is a genome-modified silkworm production kit. A kit of the present aspect comprises an expression vector necessary to produce, by a genome-editing technology, a genome-modified silkworm that produces a chimeric Fib protein comprising a silkworm-derived Fib protein and a Fib protein derived from another silk-spinning insect, a bagworm in particular. The kit of the present aspect can simply produce a genome-modified silkworm that produces the chimeric Fib protein.

### 1-2. Definition of Terms

The following terms frequently used for the present invention are defined.

The term "silk-spinning insect" as used herein collectively refers to an insect having a silk gland and being capable of spinning a silk thread. Specifically, the insect mainly refers to a species, such as in Lepidoptera, Hymenoptera, Neuroptera, or Trichoptera, the species being capable of spinning for nest building, cocoon spinning, or migration in the larval stage. In the case of the order Lepidoptera, the families Bombycidae, Saturniidae, Brahmaeidae, Eupterotidae, Lasiocampidae, Psychidae, Archtiidae, Noctuidae, and the like, which are capable of spinning a large amount of silk thread, are preferable as the silk-spinning insects according to this specification.

The term "another silk-spinning insect" as used herein refers to a silk-spinning insect other than *Bombyx mori.* The species of another silk-spinning insect is not limited. Additionally, without being limited to one species of insect, a plurality of species of insects may be used. Without limitation, another silk-spinning insect is preferably a Psychidae insect (bagworm). Unless otherwise specified, a "silkworm", which is the name in the larval stage, is herein used as the term having the same meaning as *Bombyx mori.*

The term "bagworm" collectively refers to a moth larva belonging to the family Psychidae. Unless otherwise specified, in the same manner as the above-described silkworm, a "bagworm", which is the name in the larval stage in which the insect spins, is herein used as the term having the same meaning as a Psychidae insect. The species of the bagworm herein is not particularly limited. For example, the bagworm may be of any species belonging to the genera *Acanthopsyche*, *Anatolopsyche*, *Bacotia*, *Bambalina*, *Canephora*, *Chalioides*, *Dahlica*, *Diplodoma*, *Eumeta*, *Eumasia*, *Kozhantshikovia*, *Mahasena*, *Nipponopsyche*, *Paranarychia*, *Proutia*, *Psyche*, *Pteroma*, *Siederia*, *Striglocyrbasia*, *Taleporia*, *Theriodopteryx*, and *Trigonodoma.* A Fib gene that has been cloned herein is a Fib H gene derived from *Eumeta japonica.* Thus, the bagworm is preferably a larva of *Eumeta*, such as *Eumeta japonica* or *Eumeta minuscula.*

The term "silk thread" as used herein refers to an insect-derived proteinous thread produced in a silk gland, and is spun by the insect in the larval or imaginal stage for the purpose of nest building, migration, anchoring, cocoon spinning, prey capture, and the like. A silk thread is usually constituted by a fibroin (Fib) protein as a main fibrous component and a sericin protein as a paste-like component covering the fibroin protein. Unless otherwise specified, when the term "silk thread" is simply used herein, it means both a silk thread spun by a silk-spinning insect, i.e., a "raw silk thread" comprising a Fib protein and a sericin protein, and a "degummed silk thread" consisting of a thready Fib protein obtained by degumming the raw silk thread to remove the sericin protein. When the term "silk thread" is simply used herein, it refers to a widely general silk thread from an unspecified insect of origin, in principle. In case of indicating a silk thread derived from a specific species of insect, the name of the organism of origin is placed before the term "silk thread", as a "silkworm silk thread" or a "bagworm silk thread".

The "silk gland" is a fistula resulting from a variation of the salivary gland, and functions to produce a Fib protein and a sericin protein, accumulate such a protein in the lumen, and then secrete the protein. Generally, paired silk glands are normally located bilaterally along the digestive tract of an insect capable of spinning a silk thread, and each silk gland is constituted by three regions: anterior, middle, and posterior silk glands. The posterior silk gland produces a Fib protein, and the middle silk gland produces a sericin protein. The Fib protein produced in the posterior silk gland transfers to the lumen of the middle silk gland, transfers, often together with a sericin protein, to the anterior silk gland, and then, is discharged as a silk thread through the head spinneret.

A genome-modified silkworm herein produces a chimeric Fib protein. A chimeric Fib gene encoding the chimeric Fib protein is a gene fused with an endogenous Fib gene of a silkworm, and in principle, is under the same expression control as the Fib gene of a silkworm. Hence, the silk gland that produces a chimeric Fib protein herein means the posterior silk gland, unless otherwise specified.

The term "fibroin (Fib) protein" refers to a protein that constitutes a fibrous component of silk thread. The protein usually refers to a full-length protein, but herein also comprises a peptide fragment. Here, the peptide fragment is preferably a functional fragment of a Fib protein. The "functional fragment" refers to a peptide that functions as a fibroin protein. In the case of a silkworm, it is known that the fibroin protein is, for example, constituted by three proteins: a fibroin H chain (Fib H) protein, a fibroin L chain (Fib L) protein, and a p25/FHX (herein often simply referred to as "p25") protein. Among these proteins, the Fib H protein is a main constituent protein of the Fib protein, and the physical properties of a silk thread are mainly attributed to the Fib H protein. Herein, the silkworm-derived Fib protein may be any of the three constituent proteins.

In this regard, when the term "Fib", "Fib H", or "Fib L" is herein used in relation to any of a gene and a protein, neither the gene nor the protein is limited to any insect of origin, in principle. On the other hand, when a gene or protein derived from a specific insect is referred to, the name of the organism of origin of the gene or the protein or the abbreviation of the gene or the protein is placed before Fib. For example, a silkworm-derived Fib gene or Fib protein is referred to as a silkworm Fib (sFib) gene or protein, and this is the same with a bagworm Fib (bFib) gene or protein. The same applies also to a Fib H gene or protein or a Fib L gene or protein.

As used herein, the term "modified fibroin H chain (modified Fib H) protein" (herein often referred to as an "m-Fib H protein") refers to a Fib H protein obtained by artificially modifying a wild-type Fib H protein. Examples of the m-Fib H comprise: a variant Fib H protein obtained by artificially allowing one or a plurality of amino acids to be added to, deleted from, and/or substituted in the amino acid sequence of a wild-type Fib H protein; and a fused Fib H protein obtained by fusing the amino acid sequences of the wild-type Fib H proteins derived from two or more different insects. The term "n amino acid(s)" (wherein n is an integer) as used herein has the same meaning as the term "n amino acid residue(s)", unless otherwise specified.

The term "chimeric Fib protein" as used herein refers to a Fib protein obtained by directly or indirectly linking two or more different full-length and/or partial Fib proteins. Examples comprise: a Fib protein in which, to an arbitrary site in the full length or part of a Fib L protein of one species, the full length or part of a Fib H protein of another species is linked directly or indirectly via an intervening peptide so as to form one polypeptide chain as a whole; a Fib protein in which, at a suitable position in the amino acid sequence of a Fib H protein of one species, the amino acid sequence of the full length or part of a Fib H protein of another species is inserted. In particular, the term "chimeric Fib protein" as used herein means a chimeric Fib protein comprising a Fib protein derived from a silkworm and a Fib protein derived from another silk-spinning insect, particularly a chimeric Fib protein comprising a silkworm-derived Fib protein and a bagworm-derived Fib protein (herein often referred to as a "silkworm/bagworm chimeric Fib protein (s/b Fib protein)"). Here, the bagworm-derived Fib protein is not limited, and is preferably an m-bFib H protein. Additionally, the silkworm-derived Fib protein is not limited, and may be any of an sFib L protein, sFib H protein, or p25 protein.

The term "fibroin (Fib) gene" as used herein refers to a gene encoding the Fib protein. When the Fib protein is not a full-length protein but a peptide fragment, the Fib gene means a gene fragment encoding the peptide fragment. Additionally, a "Fib H gene", "Fib L gene", and "p25 gene" refers to a gene encoding Fib H, Fib L, and p25 respectively.

As used herein, the term "modified fibroin H chain (modified Fib H) gene" (herein often referred to as an "m-Fib H gene") refers to a gene encoding m-Fib H. Thus, the term "m-bFib-H gene" refers to a gene encoding a modified Fib-H protein based on a bagworm-derived Fib-H. The present aspect is directed to this m-bFib-H gene. The term "Fib H gene" as used herein is regardless of the insect of origin, in principle, as with the protein. On the other hand, to refer to a Fib H gene derived from a specific insect, the name of the organism of origin of the gene is placed before Fib H, as in a silkworm Fib H (sFib H) gene and a bagworm Fib H (bFib H) gene.

The term "chimeric Fib gene" as used herein refers to a gene encoding the chimeric Fib protein. A genome-modified silkworm according to the present invention comprises this chimeric Fib gene in its genome.

The "chimeric silk thread" as used herein refers to a silk thread spun by a genome-modified silkworm comprising the chimeric Fib gene. The constituents of the chimeric silk thread except for the chimeric Fib protein are all from silkworm-derived silk thread. For example, when the chimeric Fib gene is a gene obtained by allowing an sFib L gene to fuse with an m-bFib H gene, a chimeric silk thread spun by a genome-modified silkworm comprising the chimeric Fib gene is a hybrid silk thread consisting of a chimeric Fib protein derived from the chimeric Fib gene, silkworm-derived sFib H, p25, and sericin. In the chimeric silk thread, part of the Fib protein as a fibrous component of the silkworm silk thread is constituted by an m-bFib H protein. Even a silk thread spun by a silkworm provides the physical properties of a bagworm silk thread by virtue of comprising modified bagworm Fib H.

The term "expression vector" as used herein refers to an expression system that comprises a gene in an expressible state, and can control the expression. The "expressible state" as used herein means that a gene comprised in an expression vector is one which has been incorporated in the vector so that the gene can be expressed in a host cell. Specifically, this means that the gene comprised is located under the control by a promoter in the expression vector.

The term "genome-modified silkworm" as used herein refers to a silkworm produced by a genome-editing technology. Herein, the genome-modified silkworm refers to a silkworm obtained by inserting (knocking-in) a bagworm-derived Fib gene in particular, preferably an m-bFib H gene, to the genome of a silkworm by a genome-editing technology.

The "genome-editing technology" is a gene-targeting technology in which an artificial DNA cleaving enzyme recognizes a specific sequence in a genome, and performs a double strand break (herein referred to as "DSB") on a DNA, whereby the insertion (knock-in) of an exogenous gene at the above-described specific position and the destruction (knock-out) of a target gene are caused. As the genome-editing technology, a TALEN method, a zinc finger nuclease (ZFN) method, a CRISPR-Cas 9 method, or the like is known. A genome-editing technology as referred to herein refers to a TALEN method, unless otherwise specified.

The "TALEN (Transcription Activator-Like Effector Nuclease) method" is a genome-editing technology to be performed with an artificial DNA cleaving enzyme, utilizing a TAL effector (TALE) protein derived from a bacteria of the genus *Xanthomonas*, which is a plant pathogenic bacteria (Cermak T, et al., 2011, Nucleic Acids Res 39: e82). The TALEN protein is a protein consisting of: a special TALE domain having repeats of DNA-binding units consisting of approximately 34 amino acid residues (LTPDQVVAIASXXGGKQALETVQRLLPVLCQDHG) illustrated by SEQ ID NO: 1; and a nuclease domain. Among these, the nuclease domain having an enzymatic activity for cleaving a DNA functions in the form of a dimer, and thus, the TALEN protein also functions in the form of a dimer consisting of: a "Left-TALEN protein" that recognizes a sense-strand DNA sequence in the upstream-side (5'-side) vicinity of the double strand break (DSB) site in a target gene: and a "Right-TALEN protein" that recognizes the antisense-strand DNA sequence in the downstream-side (3'-side) vicinity of the DSB site. In the DNA-binding unit constituting the TALE domain, the amino acid residues at position 12 and position 13 from the N-terminal in SEQ ID NO: 1 are variable. One set of two amino acids can specifically recognize each of four kinds of bases (A: adenine, G: guanine, C: cytosine, and T: thymine) constituting DNA. For example, the amino acid residues at positions 12 and 13 recognize adenine, guanine or adenine, cytosine, and thymine when the amino acid residues are N-I, N-N, H-D, and N-G respectively. The number of repeats of the DNA-binding units can vary in accordance with the base length of a target base sequence. In accordance with an arbitrary DNA sequence ranging in the direction from 5' toward 3' in a genome, the binding units that recognize the respective bases are linked to produce a TALE domain, with which a nuclease domain is fused to form a nuclease monomer (a Left-TALEN protein) that binds to the arbitrary DNA sequence in the genome. A TALEN region bound to the complementary strand of the genome at a suitable interval (15 to 30 bases) from this 5'-side TALEN binding region toward the 3' side is produced in the same manner to form another nuclease monomer (a Right-TALEN protein). Allowing these to simultaneously work as a dimer enables gene-targeting that cleaves an arbitrary DNA sequence as a target.

A "PITCh (Precise Integration into Target Chromosome) method" is a knock-in technology utilizing microhomology-mediated end joining (herein referred to as "MMEJ") (Nakade S., et al., 2014, Nature Comm., 5, 5560). MMEJ is a DNA-repair pathway that repairs a DNA by linking short 5 to 25 bp homologous sequences (microhomology) common to both ends generated by DSB. Generally, an exogenous gene is knocked-in by an HR method utilizing a mechanism of homologous recombination (HR) that makes repairs using a sister chromatid as a template. However, knock-in of an exogenous gene by an HR method requires a very long sequence as long as 800 to several thousand bp. Additionally, the frequency of occurrence is markedly different depending on the biological species, and thus, the biological species that efficiently allows knock-in is limited. A PITCh method utilizing MMEJ is not selective about the biological species to be used, and is a method that allows knock-in more efficiently than a technology utilizing conventional HR.

The term "TAL-PITCh method" (or TALEN-PITCh method) as used herein is a method using TALEN as a genome-editing tool in a PITCh method.

The term "promoter" as used herein is a gene-expression-regulating region that can control the expression of a gene (subject gene) located under the control by the promoter.

### 1-3. Constitution

A genome-modified silkworm production kit according to the present aspect comprises a Left-TALEN expression vector, a Right-TALEN expression vector, and a donor vector as essential constituents. In principle, these three constituents are used as one set.

The vectors comprised in the genome-modified silkworm production kit do not need to be separate vectors, subject to comprising the respective constituents. The constitution may be such that two or three vectors are combined as one vector. Examples of the constitution comprise: a Left/Right-TALEN expression vector as one combination of a Left-TALEN expression vector and a Right-TALEN expression vector; and one vector comprising all the three vectors.

Below, the constitution of each of the vectors will be specifically described.

### 1-3-1. Left-TALEN Expression Vector

The term "Left-TALEN expression vector" as used herein refers to an expression vector comprising a Left-TALEN region and a promoter as essential constituents. In a genome-modified silkworm production kit according to the present invention, the Left-TALEN expression vector may be introduced into a silkworm cell used for genome editing, and express a Left-TALEN protein in vivo, or may be used for in-vitro transcription to prepare a Left-TALEN mRNA.

### (Constitution)

Below, each of the constituents of the Left-TALEN expression vector will be described.

### (1) Left-TALEN Region

The term "Left-TALEN region" as used herein is a region encoding a Left-TALE domain and a nuclease domain located downstream thereof, and encodes the above-described Left-TALEN protein. Below, the Left-TALE domain and the nuclease domain that are constituents of the Left-TALEN region will be described.

### (1-1) Left-TALE Domain

The term "Left-TALE domain" as used herein is a DNA-binding domain in TALEN, and constituted by an amino acid sequence comprising the repetitive sequences of a DNA-binding unit consisting of 34 amino acids and shown in the above-described SEQ ID NO: 1. The number of the repeats is equal to the number of the bases of the 5'-side TALE binding region in the sFib gene. For example, when the number of the bases of the 5'-side TALE binding region is 15, the number of repeats of the DNA-binding unit is 15. The 5'-terminal side of the base sequence encoding the Left-TALE domain may comprise the N-terminal domain, and additionally, the 3'-terminal side may comprise the C-terminal domain. The 5' side of the 5'-side TALE binding region may comprise thymine (T).

The term "5'-side TALE binding region" as used herein is a region consisting of a specific base sequence in the sFib gene in the genome of a silkworm, and is a target region which the Left-TALEN protein according to the present invention specifically recognizes and binds to via the Left-TALE domain. The sequence and length of the bases constituting the 5'-side TALE binding region are as follows: an arbitrary base sequence of 11 bases to 31 bases, 12 bases to 30 bases, 13 bases to 28 bases, 14 bases to 26 bases, or 15 bases to 25 bases selected in the sequence of the 5 to 55 bases upstream from a desired insertion site of the bFib gene, i.e., from the DSB site in the sFib gene in the genome of a silkworm. Accordingly, the base sequence of the 5'-side TALE binding region may be designed suitably in accordance with the DSB site.

### (1-2) Nuclease Domain

The term "nuclease domain" as used herein is a domain consisting of an endonuclease linked downstream of the Left-TALE domain or the below-described Right-TALE domain, or consisting of an active domain of the endonuclease. The Left-TALEN protein and the Right-TALEN protein that are bound to the 5'-side TALE binding region and the 3'-side TALE binding region respectively in the Fib gene of a silkworm via the Left-TALE domain or the Right-TALE domain cleave a target DSB site by the endonuclease activity possessed by the respective nuclease domains. The endonuclease is not particularly limited to any kind, subject to having no specific recognition sequence, and having a DSB activity to an arbitrary sequence. Examples comprise an endonuclease obtained by removing the base sequence-recognizing domain from FokI.

### (2) Promoter

The term "promoter" as used herein refers to a transcriptional regulatory region that controls the downstream gene expression. A promoter to be used in the present invention may be derived from any gene, subject to being a promoter capable of working in an environment in which a downstream gene is transcribed. The phrase "being capable of working" refers to being capable of performing a promoter function, and transcribing a subject gene and the like.

The promoter is not limited to any kind. In the case of the intracellular transcription of a silk-spinning insect, examples of the promoter comprise: a ubiquitously expressible constitutive expression promoter that controls the expression of a housekeeping gene; a constitutively active promoter; a stage-specific active promoter; a site-specific promoter; and an expression-inducing promoter. Without limitation, a constitutive expression promoter or a stage-specific active promoter is preferable. Examples of the constitutive expression promoter comprise an actin promoter. Additionally, examples of the expression-inducing promoter comprise a hsp70 promoter. On the other hand, examples of the in-vitro transcription promoter utilizing a bacteriophage-specific RNA polymerase or the like comprise an SP6 promoter, T3 promoter, and T7 promoter.

Specific examples of the base sequence of the in-vivo promoter derived from a silk-spinning insect comprise a silkworm-derived actin promoter comprising the base sequence of SEQ ID NO: 2.

### 1-3-2. Right-TALEN Expression Vector

The term "Right-TALEN expression vector" as used herein refers to an expression vector that functions in the form paired with the Left-TALEN expression vector, and comprises a Right-TALEN region and a promoter as essential constituents. Accordingly, the basic constitution is in accordance with the constitution of the Left-TALEN expression vector. In a genome-modified silkworm production kit according to the present invention, the Right-TALEN expression vector may be introduced into a silkworm cell used for genome editing, and express a Right-TALEN protein in vivo, or may be used for in-vitro transcription to prepare a Right-TALEN mRNA.

### (Constitution)

Below, each of the constituents of the Right-TALEN expression vector will be described.

### (1) Right-TALEN Region

The term "Right-TALEN region" as used herein is a region encoding a Right-TALE domain and a nuclease domain located downstream thereof, and encodes the above-described Right-TALEN protein. The basic constitution is in accordance with the constitution of the Left-TALEN region in the Left-TALEN expression vector. Accordingly, the description of the same constituents as of the Left-TALEN region is omitted here, and the different constituents will mainly be described.

### (1-1) Right-TALE Domain

The term "Right-TALE domain" as used herein is a DNA-binding domain in TALEN, and constituted by the repetitive sequences of a DNA-binding unit consisting of 34 amino acids and shown in the above-described SEQ ID NO: 1. The basic constitution is in accordance with the constitution of the Left-TALE domain, but the Right-TALE domain targets the 3'-side TALE binding region in the sFib gene, and the number of repeats is equal to the number of the bases of the 3'-side TALE binding region in the sFib gene. At the 5'-side of the 3'-side TALE binding region recognized by the Right-TALE domain, thymine (T) is usually located in the same manner as at the 5'-side of the 5'-side TALE binding region recognized by the Left-TALE domain. However, this thymine does not necessarily have to be located.

The term "3'-side TALE binding region" as used herein refers to a region consisting of a specific base sequence in the Fib gene in the genome of a silkworm, and is a target region which the Right-TALEN protein according to the present invention specifically recognizes and binds to via the Right-TALE domain. The sequence and length of the bases constituting the 3'-side TALE binding region are as follows: an arbitrary base sequence of 11 bases to 31 bases, 12 bases to 30 bases, 13 bases to 28 bases, 14 bases to 26 bases, or 15 bases to 25 bases selected in the sequence of the 5 to 55 bases downstream from a desired insertion site of the bFib gene, i.e., from the DSB site, in the sFib gene in the genome of a silkworm. Accordingly, the base sequence of the 3'-side TALE binding region may be designed suitably in accordance with the DSB site. The 3'-side TALE binding region and the 5'-side TALE binding region have the DSB site sandwiched therebetween, and function in the form of one set of two regions. In the base sequence and/or the base length, the 3'-side TALE binding region may be the same as or different from the 5'-side TALE binding region.

### (1-2) Nuclease Domain

The constitution of the nuclease domain is in accordance with the constitution of the nuclease domain in the Left-TALEN expression vector. The nuclease domain in the Right-TALEN expression vector may be the same as or different from the Left-TALEN expression vector, and is preferably the same. In principle, the kind of the nuclease is the same as the kind of the nuclease in the Left-TALEN expression vector.

### (2) Promoter

The constitution of the promoter is in accordance with the constitution of the promoter in the Left-TALEN expression vector. The promoter in the Right-TALEN expression vector may be the same as or different from the Left-TALEN expression vector, and is preferably the same.

### 1-3-3. Donor Vector

The term "donor vector" as used herein is a vector that is inserted in the genome of a silkworm as a host by a genome-editing technology to express a Fib H gene derived from another silk-spinning insect.

### (Constitution)

The donor vector comprises a 5'-side TALE corresponding region, a 3'-side TALE corresponding region, a 5'-side homologous region, a 3'-side homologous region, and a fibroin gene of another silk-spinning insect as constituents. Below, each of the constituents will be described.

### (1) 5'-side TALE Corresponding Region

The term "5'-side TALE corresponding region" as used herein is a target region which the TALEN protein specifically recognizes and binds to via the TALE domain. When inserted, all or part of the donor vector needs to be linear to be knocked-in the genome of a silkworm by a genome-editing technology. The 5'-side TALE corresponding region has substantially the same function as the TALE binding region in the genome, and is located as one of a pair of the TALE corresponding regions between which the DSB site in the donor vector is sandwiched, wherein the location is upstream in the direction of insertion of the fibroin gene to be knocked-in from another silk-spinning insect. Accordingly, the 5'-side TALE corresponding region is not necessarily the same as the base sequence of the 5'-side TALE binding region on the genome side. For example, differently from the 5'-side TALE binding region to be recognized by the Left-TALEN protein, the 5'-side TALE corresponding region may have a sequence to be recognized by any of the Left-TALEN protein and the Right-TALEN protein. In this regard, when located on the coding sequence of a protein to be expressed by a silkworm, or located in a control sequence of a promoter or the like inserted as an optional constituent, the 5'-side TALE corresponding region is restricted by the sequence.

The base length of the base sequence constituting the 5'-side TALE corresponding region may be 11 bases to 31 bases, 12 bases to 30 bases, 13 bases to 28 bases, 14 bases to 26 bases, or 15 bases to 25 bases.

### (2) 3'-side TALE Corresponding Region

As with the 5'-side TALE corresponding region, the term "3'-side TALE corresponding region" as used herein is a target region which the TALEN protein specifically recognizes and binds to via the TALE domain. The 3'-side TALE corresponding region has the same function as the TALE binding region in the genome, and is located in a pair of the TALE corresponding regions between which the DSB site in the donor vector is sandwiched, wherein the location is downstream in the direction of insertion of the fibroin gene to be knocked-in from another silk-spinning insect. Accordingly, the 3'-side TALE corresponding region is not necessarily the same as the base sequence of the 3'-side TALE binding region on the genome side. For example, differently from the 3'-side TALE binding region to be recognized by the Right-TALEN protein, the 3'-side TALE corresponding region may have a sequence to be recognized by any of the Left-TALEN protein and the Right-TALEN protein.

The base length of the base sequence constituting the 3'-side TALE corresponding region may be 11 bases to 31 bases, 12 bases to 30 bases, 13 bases to 28 bases, 14 bases to 26 bases, or 15 bases to 25 bases.

In the donor vector, the 3'-side TALE corresponding region and the 5'-side TALE corresponding region function in the form of one set of two regions.

### (3) 5'-side Homologous Region

The term "5'-side homologous region" as used herein is a region needed to knock-in the in bFib H gene in the donor vector at a predetermined position in the genome of a silkworm, and consists of a base sequence the same as or homologous with the 5'-side base sequence at the insertion site, i.e., the DSB site in the genome of a silkworm. The base sequence of the 5'-side homologous region is not limited to any length, and is within the reach of the nuclease activity of a dimer consisting of the Left-TALEN protein and/or the Right-TALEN protein bound to the 5'-side TAL corresponding region or the 3'-side TALE corresponding region. The base length of the 5'-side homologous region depends on the TALEN to be used, and may be, for example, 5 to 15 bases, 6 to 14 bases, 7 to 13 bases, 8 to 12 bases, or 9 to 11 bases. The base length is preferably 8 to 10 bases. The base length of the below-described 3'-side homologous region is the same as or equal to the base length of the corresponding 5'-side homologous region, in principle. Thus, the base length of a spacer sequence consisting of the 5'-side homologous region and the 3'-side homologous region that are between the 5'-side and 3'-side TALEN corresponding regions is twice as long as the base length of the 5'-side homologous region, and is, for example, 10 to 30 bases, 12 to 28 bases, 14 to 26 bases, 16 to 24 bases, or 18 to 22 bases. The base length is preferably 16 to 20 bases.

All or part of the donor vector is knocked-in the genome of a silkworm via the 5'-side homologous region and the below-described 3'-side homologous region by homology-directed repair (HDR), microhomology-mediated end joining (MMEJ), or nonhomologous end joining (NHEJ) between the DSB ends of the genome and the donor.

### (4) 3'-side Homologous Region

The term "3'-side homologous region" as used herein is a region needed to knock-in the in bFib H gene in the donor vector at a predetermined position in the genome of a silkworm, and functions in the form paired with the 5'-side homologous region. The 3'-side homologous region consists of a base sequence the same as or homologous with the base sequence flanked by the insertion site in the genome of a silkworm, i.e., the 3' side of the DSB site. The base sequence of the 3'-side homologous region is not limited to any length, and is within the reach of the nuclease activity of a dimer consisting of the Left-TALEN protein and/or the Right-TALEN protein bound to the 5'-side TALE corresponding region and the 3'-side TALE corresponding region. The base length may be, for example, 5 to 15 bases, 6 to 14 bases, 7 to 13 bases, 8 to 12 bases, or 9 to 11 bases. The base length is preferably 8 to 10 bases.

### (5) Fibroin Gene of Another Silk-spinning Insect

The "fibroin gene of another silk-spinning insect" is a gene encoding the Fib protein of a silk-spinning insect other than a silkworm. The Fib gene may be the full length or part of a single species of Fib gene, or may be any of the Fib genes formed by linking a plurality of species of Fib genes, as follows: linking the full-length base sequences; linking the full-length base sequence and a partial base sequence; and linking partial base sequences. Additionally, the Fib gene of the another silk-spinning insect may be any of the Fib H gene, the Fib L gene, and the p25 gene. The Fib gene is preferably the Fib H gene. Additionally, the another silk-spinning insect is not limited to any species, and is preferably a bagworm. Accordingly, the bagworm fibroin H chain gene is particularly preferable as the Fib gene of the another silk-spinning insect, without limitation.

The term "bagworm fibroin H chain gene (bFib H gene)" is a gene encoding the bagworm fibroin H chain protein. Below, the constitution of the bagworm fibroin H chain protein will be described.

The "bagworm fibroin H chain protein (bFib H protein)" may be any of a wild-type fibroin H chain protein and a modified fibroin H chain protein. The protein is preferably a modified fibroin H chain protein.

The "modified bagworm fibroin H chain protein (m-bFib H protein)" herein comprises m-Fib H in which the sites insufficient in the information on the amino acid sequence are complemented with reference to the information on the amino acid sequence of the corresponding silkworm Fib H on the basis of the amino acid sequence of part of the Fib H protein of *E. japonica*, wherein this amino acid sequence has been revealed by a transcriptomic analysis using a next-generation DNA sequencer.

The m-bFib H protein herein is not limited to any constitution. For example, the constitution comprises, as basic constituents, the N-terminal region, central region, and C-terminal region in this order from the N-terminal side, as shown in Figure 2A.

The term "N-terminal region" as used herein refers to an optional amino acid region located on the N-terminal side of the below-mentioned central region in the amino acid sequence constituting the m-bFib H protein.

The term "C-terminal region" as used herein refers to an optional amino acid region located on the C-terminal side of the below-mentioned central region in the amino acid sequence constituting the m-bFib H protein.

The "central region" herein is a region that exhibits the physical properties of an m-bFib H protein, and is constituted by three or more same and/or different repeat units that are linked.

The "repeat unit" herein is a unit that consists of a full length of 120 to 178 amino acids, and comprises a plurality of G/A units and one alanine cluster.

The "G/A unit" is a unit consisting of two amino acid residues, i.e., a glycine (Gly: G) residue and an alanine (Ala: A) residue, and is constituted by glycine residue-alanine residue (GA) or alanine residue-glycine residue (AG). The majority of the repeat unit is constituted by G/A units, and the number of G/A units comprised in one unit is 30 units or more, 35 units or more, or 40 units or more, or 60 units or less, 55 units or less, or 50 units or less.

The "alanine cluster" (herein often referred to as an "Ala cluster") is a subunit consisting of consecutive alanine (Ala) residues, and is comprised on the N-terminal side in the repeat unit. One Ala cluster comprises 15 to 25 alanine residues, and other than the alanine residues, one glutamic acid or glutamine may be comprised in the central portion of the Ala cluster (for example, position 10 from the N-terminal side of the Ala cluster). Specific examples of the Ala cluster in the m-bFib H protein comprise the amino acid sequences of SEQ ID NOs: 3 and 4 (AAAAAAAAAEAAAAAAAAAAAA and AAAAAAAAAQAAAAAAAAA respectively).

Furthermore, the repeat unit may comprise a non-G/A portion comprising amino acid residues that consist of 5 to 7 amino acids and other than a glycine residue and an alanine residue. The non-G/A portion is not limited to any amino acid sequence. Specific examples of the amino acid sequence of the non-G/A portion comprise the amino acid sequences of SEQ ID NO: 5 (YGSALNS), SEQ ID NO: 6 (SALNS), and SEQ ID NO: 7 (TSVVYV) that consist of serine (Ser: S) residues, valine (Val: V) residues, and tyrosine (Tyr: Y) residues.

The amino acid sequence constituting the repeat unit of the m-bFib H protein herein is not limited to any particular amino acid sequence, subject to satisfying the conditions for each of the above-described constituents. Specific examples comprise, but are not limited to, the amino acid sequences of SEQ ID NOs: 8 to 16 listed in Table 1.

**[Table 1]**

| No. | Amino Acid Sequence of Repeat Unit of m-bFib H Protein | SEQ ID NO: |
|---|---|---|
| 1 | AAAAAAAAQAAAAAAAAAGSGAGAGRVGAYGPYGGLASAGAGGAGGAGGAGGYGGASV VYVGGGGAGAGAGSGAGAGSGAGAGAGSGAGAGGAGAAAGAGAGAGSGAGSGSGAGAGA GSGAGAGSGAGAGAGAGSGAAGGAGAGAGAGAG | 8 |
| 2 | AAAAAAAAEAAAAAAAAAAAAAGSGAGAGGAGGYGAGAGAGAGAGGAAGAGGAGGAG GAGGYGGASAGAGGAGGAGGAGGYGGAGVVYVSAGGAGAGAGSGAGAGSGAGAGAGSGA GAGGAGAAAGAGAGAGSGAGSGSGAGAGAGSGAGAGSGAGAGAGAGSGAAGGAGAGAGA G | 9 |
| 3 | AAAAAAAAAEAAAAAAAAAAAAAGSGAGAGGAGGYGAGAGAGAGAGGASGGYGSYGSG VAAGAGAGAGVGGSRGAGVGAGVGAGYGSALNSGAGAGAGAGAGAGGAAGAGAGAGAGA GSGAAGGAGAGAGAG | 10 |
| 4 | AAAAAAAAEAAAAAAAAAAAAAGGAGGYGAGAGAGAGAGAGTSAGAGGAGGAGGAGGYG GAGVVYVSAGGAGAGAGSGAGAGSGAGAGAGSGAGAGGAGAAAGAGAGAGSGAGSGSGA GAGAGSGAGAGSGAGAGAGAGSGAAGGAGAGAGAG | 11 |
| 5 | AAAAAAAAEAAAAAAAAAAAAAGGAGGYGAGAGAGAGAGTSVVYVSAGGAGAGAGSG AGAGSGAGAGAGSGAGAGGAGAAAGAGAGAGSGAGSGSGAGAGAGSGAVAGSGAGSGAA GGAGAGAGAG | 12 |
| 6 | AAAAAAAAEAAAAAAAAAAAAAGSGAGAGGAGGYGAGAGAGAGAGVGGAGGAGGAGGAG GAGGYGGAGVVYVSAGGAGAGAGSGAGAGSGAGAGAGSGAGAGGAGAAAGAGAGAGSGA GSGSGAGAGAGSGAVAGSGAGSGAAGGAGAGAGAG | 13 |
| 7 | AAAAAAAAAEAAAAAAAAAAAAAGSGAGAGGAGGYGAGAGAGAGAGGASGGYGSYGSG VAAGAGAGAGVGGSRGAGVGAGVGAGYGSALNSGAGAGAGAGAGGAAGAGAGAGAGA GAGAG | 14 |
| 8 | AAAAAAAAEAAAAAAAAAAAAAGSGAGAGGAGGYGAGAGAGAGAGGAAGAGGAGGAG GAGGYGGASVVYVSAGGAGAGAGSGAGAGSGAGAGAGSGAGAGGAGAAAGAGAGAGSGA GSGSGAGAGAGSGAVAGSGAGSGAAGGAGAGAGAG | 15 |
| 9 | AAAAAAAAEAAAAAAAAAAAAAAGSGAGAGGAGGYGAGAGAGAGAGAGGAAGAGGAGGAG GAGGYGGTSVVYVGGGGAGAGAGSGAGAGSGAGAGAGSGAGAGGAGAAAGAGAGAGSGA GSGSGAGAGAGSGAGAGSGAGAGAGAGSGAAGGAGAGAGAGAG | 16 |

The repeat units in the m-bFib H protein are mutually linked directly or via any linker sequence consisting of another 1 to 30 amino acids, 1 to 20 amino acids, or 1 to 10 amino acids.

Specific examples of the base sequence of the modified bagworm fibroin H chain gene encoding the repeat unit of the above-described m-bFib H protein comprise the base sequences of SEQ ID NOs: 17 to 25 listed in Table 2 below, which encode the repeat unit Nos. 1 to 9 respectively listed in Table 1.

**[Table 2]**

| No. | Base Sequence Encoding Repeat Unit of m-bFib H Protein | SEQ ID NO: |
|---|---|---|
| 1 | gctgcagccgccgctgcggcagcagcacaagcagcagccgcagctgctgccgccgcagg tagtggcgctggtgctggacgagtcggagcttacggaccctacggaggtttagcaagcg ctggtgctggtggtgctggcggagccggtggtgcaggtggatacggtggtgcaagtgtc gtctacgtaggtggcggaggagccggagcaggtgccggttcaggcgccggtgctggatc gggtgccggagcaggagctggatcaggtgcaggagcaggtggagcaggagccgcagccg gagctggtgctggtgctggttcaggtgcaggatccggttcaggtgcgggcgctggagca ggttccggagcaggagcaggttccggtgctggtgcaggagcaggagctggctcaggtgc agcaggtggagcaggagcaggagctggcgctggcgctggc | 17 |
| 2 | gctgcagccgcagccgcagcagcagcagaagcggcagccgcagctgccgccgccgctgc cgccgcaggtagtggagcaggcgctggaggagccggaggctacggagcgggagctggag caggtgccggtgcaggtgctggcggcgctgctggtgctggtggtgctggcggagccggt ggtgcaggtggatacggtggtgctagcgcgggtgcgggtggcgcgggtggcgcgggtgg cgcgggtggctacggtggcgcgggtgtggtttatgttagcgcgggtggcgcgggcgcgg gtgcgggtagcggtgcgggtgcgggcagcggtgccggcgcgggcgcgggcagcggcgcg ggagcgggtggcgcgggcgctgcggcgggtgcgggcgcgggtgcgggcagcggagcggg atccggtagcggcgctggcgctggtgcgggtagcggagctggcgcgggcagcggtgcgg gtgcgggtgcgggcgcgggtagcggtgcggcgggtggcgcgggagcaggcgcgggtgcg ggt | 18 |
| 3 | gccgctgccgccgcggcggcggcggaggcagctgccgcagcagctgccgcagcggc ggcggcgggtagcggtgcgggcgcgggtggcgcgggtggctacggggctggcgctggtg ctggtgctggtgctggcgcgggtggcgcgagcggtggctacggtagctatggcagcggt gtggcggcgggtgctggcgcgggcgcgggtgttggtggcagccgtggcgcgggtgtggg tgcgggtgttggtgcgggttatggcagcgcgctgaatagcggcgctggagcaggtgctg gtgccggcgctggcgcgggtggcgcggctggcgctggtgccggtgctggtgcaggagct ggcagcggagctgcgggtggcgccggagctggagctggcgcggga | 19 |
| 4 | gctgccgccgctgccgcagctgcggcggaagcagctgccgccgctgctgctgctgc tgccgcggtggcgcggtggctacggagctggagccggcgcaggcgctggtgccggcg cgggtactagcgcggtgcggtggcgcggtggcgcggtggcgcggtggctacggt ggcgcggtgtggtttatgttagcgcggtggcgcggcgcggtgcggtagcggtgc gggtgcggcagcggtgccggcgcggcgcggcagcggcgcgggagcgggtggcgcgg gcgctgcggcgggtgcggcgcgggtgcggcagcggagcgggatccggtagcggcgct ggcgctggtgcgggtagcggagctggcgcggcagcggtgcgggtgcggtgcggcgc gggtagcggtgcggcgggtggcgcgggagcaggcgcgggtgcgggt | 20 |
| 5 | gctgccgccgctgccgcagctgcggcggaagcagctgccgccgctgctgctgctgc tgccgcgggtggcgcgggtggctacggagctggagccggcgcaggcgctggtgccggcg cgggtactagcgtcgtctacgtaagtgctggaggagccggagcaggtgccggttcaggc gccggtgctggatcgggtgccggagcaggagctggatcaggtgcaggagcaggtggagc aggagccgcagccggagctggtgctggtgctggttcaggtgcaggatccggttcaggtg cgggcgctggagcaggttccggagcagtagcaggttccggtgctggttcaggtgcagca ggtggagcaggagctggcgctggcgctggc | 21 |
| 6 | gctgcagccgcagccgcagcagcagcagaagcggcagccgcagctgccgccgccgctgc cgccgcaggtagtggagcaggcgctggaggagccggaggctacggagcgggagctggag caggtgccggagcaggtgttggcggcgctggtggtgctggtggtgctggcggagccggt ggtgcaggtggatacggtggtgcaggcgtcgtctacgtaagtgctggaggagccggagc aggtgccggttcaggcgccggtgctggatcgggtgccggagcaggagctggatcaggtg caggagcaggtggagcaggagccgcagccggagctggtgctggtgctggttcaggtgca ggatccggttcaggtgcgggcgctggagcaggttccggagcagtagcaggttccggtgc tggttcaggtgcagcaggtggagcaggagctggcgctggcgctggc | 22 |
| 7 | gctgcagccgcagccgcagcagcagcagaagcggcagccgcagctgccgccgccgctgc cgccgcaggtagtggagcaggcgctggaggagccggaggctacggagcgggagctggag caggtgccggtgcaggtgctggcggcgcatctggaggctacggttcttacggatcggga gttgcagcaggtgccggtgcaggagctggtgttggtggtagcaggggagcaggtgttgg cgctggtgttggtgctggttatggctccgcattgaattcaggagccggtgccggagcag gtgctggtgctggagctggtggtgctgcaggagctggtgctggtgcaggagcaggagct ggcgctggagctgga | 23 |
| 8 | gctgcagccgcagccgcagcagcagcagaagcggcagccgcagctgccgccgccgctgc cgccgcaggtagtggagcaggcgctggaggagccggaggctacggagcgggagctggag caggtgccggtgcaggtgctggcggcgctgctggtgctggtggtgctggcggagccggt ggtgcaggtggatacggtggtgctagcgtcgtctacgtaagtgctggaggagccggagc aggtgccggttcaggcgccggtgctggatcgggtgccggagcaggagctggatcaggtg caggagcaggtggagcaggagccgcagccggagctggtgctggtgctggttcaggtgca ggatccggttcaggtgcgggcgctggagcaggttccggagcagtagcaggttccggtgc tggttcaggtgcagcaggtggagcaggagctggcgctggcgctggc | 24 |
| 9 | gctgcagccgcagccgcagcagcagcagaagcggcagccgcagctgccgccgccgctgc cgccgcaggtagtggagcaggcgctggaggagccggaggctacggagcgggagctggag caggtgccggtgcaggtgctggcggcgctgctggtgctggtggtgctggcggagccggt ggtgcaggtggatacggtggtactagcgtcgtctacgtaggtggcggaggagccggagc aggtgccggttcaggcgccggtgctggatcgggtgccggagcaggagctggatcaggtg ctggagcaggtggagcaggagccgcagccggagctggtgctggtgctggttcaggtgca ggatccggttcaggtgcgggcgctggagcaggttccggagcaggagcaggttccggtgc tggtgcaggagcaggagctggttcaggtgcagcaggtggagcaggagcaggagctggcg ctggcgctggc | 25 |

### (Order of Location)

When based on the 5' to 3' direction in the sense strand of the fibroin gene that is in the another silk-spinning insect, and comprised in the donor vector, the constituents in the donor vector are located, as follows: the 5'-side homologous region and the 3'-side homologous region are between two TALE corresponding regions, and are flanked by each other with the DSB site sandwiched between the homologous regions. The 3'-side homologous region and the 5'-side homologous region are located in this order from the 5' side. The two TALE corresponding regions are located as the 5'-side TALE corresponding region and the 3'-side TALE corresponding region in this order on the basis of the 5' to 3' direction, and may each have a sequence recognized by any of the Left-TALEN protein and the Right-TALEN protein. Specifically, on the basis of the 5' to 3' direction, the 5'-side TALE corresponding region, the 3'-side homologous region, the 5'-side homologous region, and the 3'-side TALE corresponding region are located in this order, for example, in the following cases: (i) in which the 5'-side TALE corresponding region and the 3'-side TALE corresponding region are recognized by the Left-TALEN protein; (ii) in which the 5'-side TALE corresponding region is recognized by the Left-TALEN protein, and the 3'-side homologous region is recognized by the Right-TALEN protein; (iii) in which the 5'-side TALE corresponding region is recognized by the Right-TALEN protein, and the 3'-side homologous region is recognized by the Left-TALEN protein; and (vi) in which the 5'-side TALE corresponding region and the 3'-side TALE corresponding region are recognized by the Right-TALEN protein.

### 2. Genome-modified Silkworm Production Method

### 2-1. Overview

A second aspect of the present invention is a genome-modified silkworm production method. The production method of the present aspect can produce a genome-edited silkworm that produces, in a silk gland, a chimeric Fib protein comprising a bagworm-derived fibroin protein and a silkworm-derived fibroin protein.

### 2-2. Method

A method of producing a genome-modified silkworm according to the present invention comprises a nucleic acid introducing process, a transformant selecting process, and a genome-inserted individual selecting process as essential processes. Below, each of the processes will be described.

### 2-2-1. Nucleic Acid Introducing Process

The "nucleic acid introducing process" is a process of introducing a Left-TALEN mRNA or Left-TALEN expression vector, a Right-TALEN mRNA or Right-TALEN expression vector, and a donor vector into an egg of a silkworm.

The respective constitutions of the Left-TALEN expression vector and the Right-TALEN expression vector are in accordance with the respective constitutions of the Left-TALEN expression vector and the Right-TALEN expression vector according to the first aspect. However, the Left-TALEN expression vector and the Right-TALEN expression vector according to the present aspect comprise a promoter that is expressed in the early embryo. Accordingly, these expression vectors express the Left-TALEN protein and the Right-TALEN protein in the early embryo.

The "Left-TALEN mRNA" and the "Right-TALEN mRNA" are mRNAs comprising the Left-TALEN region and the Right-TALEN region respectively. The respective constitutions of the Left-TALEN region and the Right-TALEN region are in accordance with the respective constitutions of the Left-TALEN region and the Right-TALEN region according to the first aspect.

The Left-TALEN mRNA or the Left-TALEN expression vector and the Right-TALEN mRNA or the Right-TALEN expression vector are not limited to any combination in the introduction into an egg of a silkworm. All of the four species may be introduced, or any arbitrarily selected three species may be introduced. Alternatively, the combination may be a combination of Left- and Right-TALEN mRNAs, a combination of Left- and Right-TALEN expression vectors, a combination of a Left-TALEN mRNA and a Right-TALEN expression vector, or a combination of a Left-TALEN expression vector and a Right-TALEN mRNA.

The constitution of the donor vector is in accordance with the constitution of the donor vector according to the first aspect.

Any method known in the art may be used as a method of introducing the expression vector and/or mRNA into an egg of a silkworm. For example, Tamura and colleagues' method (Tamura T. et al., 2000, Nature Biotechnology, 18, 81-84) can be used. Specifically, a dosing solution is prepared by diluting the expression vector or mRNA in a solvent such as water or a buffer to an appropriate concentration.

A nucleic acid is introduced into a fertilized egg of a silkworm by microinjection within 6 hours immediately after oviposition. Without limitation, the injection is generally performed with a special injection device utilizing air pressure. For example, a method in JP1654050B2 or the Tamura and colleagues' method (Tamura T. et al., 2007, J Insect Biotechnol Sericol, 76, 155-159) can be used. The amount of the nucleic acid to be introduced is not particularly limited. The amount may be suitably determined in accordance with the kind, nature, and purpose of the nucleic acid. The amount is usually 1 nL to 5 nL.

### 2-2-2. Transformant Selecting Process

The "transformant selecting process" is a process of selecting a transformant(s) comprising a donor vector from a silkworm(s) after the nucleic acid introducing process. A method of selecting the transformant is not limited, subject to being a method known in the art. For example, when the donor vector comprises a marker gene, a transformant of interest can be easily selected on the basis of the expression of the marker gene.

The "marker gene" is a polynucleotide consisting of a base sequence encoding a marker protein, which is also called a selection marker.

The "marker protein" is a protein that, by virtue of the expression of a marker gene, can add a new trait not possessed by a host silkworm. Examples of the marker protein comprise enzymes, fluorescent proteins, chromogenic proteins, and light-emitting proteins. On the basis of the activity of the marker protein, a transformant holding the nucleic acid introduced therein can be distinguished. The phrase "on the basis of the activity" means "on the basis of the detected activity level". In the detection of the activity, the activity of a marker protein itself may be detected directly or indirectly via a metabolite generated by the activity of the protein. The detection may be any one selected from chemical detection (comprising detections based on enzymatic reactions), physical detection (comprising detections based on behavior analyses), or sensory detection by a detector (comprising detections based on a detector's visual, tactile, olfactory, auditory, or gustatory sense).

The marker protein is not limited to any particular type as long as the activity of the marker protein can be detected by any method known in the art. Preferably, the marker protein in the detection has low invasiveness to a host holding a transformant distinguishing marker, i.e., to a transformant. Examples of such a marker protein comprise fluorescent proteins, chromogenic proteins, luminescent proteins, secretory proteins, and proteins controlling external morphology. Fluorescent proteins and chromogenic proteins are particularly suitable because these proteins can be visually detected without affecting the external morphology of transformants under specific conditions, and are thus much less invasive to transformants, which in turn enables easy identification and selection of transformants.

The term "fluorescent protein" as used herein refers to a protein that emits fluorescence at a specific wavelength when irradiated with excitation light having a specific wavelength. The fluorescent protein may be either natural or unnatural. Additionally, neither the wavelength of the excitation light nor the wavelength of the fluorescent light is particularly limited. Specific examples of the fluorescent protein comprise CFP, RFP, DsRed (comprising derivatives such as DsRed-monomer), YFP, PE, PerCP, APC, and GFP (comprising derivatives such as EGFP).

The term "chromogenic protein" as used herein refers to a protein, usually an enzyme, which is involved in pigment biosynthesis. The term "pigment" as used herein refers to a low-molecular-weight compound or peptide that can color a transformant. The pigment is not limited to any particular type. The pigment is preferably a pigment that contributes to the external body color of an individual. Examples of the pigment comprise melanin pigments (comprising dopamine-melanin), ommochrome pigments, and pteridine pigments.

### 2-2-3. Genome-inserted Individual Selecting Process

The "genome-inserted individual selecting process" is a process of selecting, from the transformant(s), an individual in which, at a target position in the genome of the silkworm, a fibroin gene of another silk-spinning insect is inserted (knocked-in). A method of verifying whether the fibroin gene of the another silk-spinning insect has been knocked-in accurately is not limited, subject to being a method known in the art. Examples comprise: a Southern hybridization method using a genomic DNA prepared from each of a transformant obtained in the transformant selecting process and a control silkworm having no nucleic acid introduced in the nucleic acid introducing process, and using a probe consisting of a base sequence specific to a fibroin gene of another silk-spinning insect; and verifying the insertion site, the direction of insertion, and the like by using a nucleic acid amplification method to amplify a region comprising the insertion site (DSB site) in the genome, and by determining the base sequence of the nucleic acid fragments obtained. The present process can select a genome-modified silkworm of interest.

### 3. Genome-modified Silkworm

### 3-1. Overview

A third aspect of the present invention is a genome-modified silkworm. A genome-modified silkworm according to the present invention comprises a bagworm-derived fibroin H chain gene in the genome of a silkworm, and can produce the bagworm fibroin H chain protein in a silk gland. A genome-modified silkworm according to the present invention can spin a chimeric silk thread comprising a chimeric fibroin protein of a silkworm-derived fibroin protein and a bagworm fibroin H chain protein.

### 3-2. Constitution

A genome-modified silkworm according to the present aspect is a silkworm obtained in a case in which a fibroin of another silk-spinning insect, comprised in a donor vector, is the m-bFib H gene in a method of producing the genome-modified silkworm according to the second aspect. This silkworm comprises, in the genome, the m-bFib H gene, preferably in the form of a chimeric Fib gene of the m-bFibe H gene and the sFib gene. In the case of the chimeric Fib gene, the insertion site of the m-bFib H gene may be in the range of from the 5' terminal to the 3' terminal (excluding the termination codon) in the coding region of the sFib gene. In this regard, the sFib gene may be any of the sFib H gene, the sFib L gene, and the sp25 gene, and is preferably the sFib H gene or the sFib L gene.

The genome-modified silkworm of the present aspect can produce the m-bFib H protein in the posterior silk gland in the larval stage, particularly the late final instar stage. In this case, it is preferable to produce a chimeric Fib protein comprising an sFib protein.

The genome-modified silkworm of the present aspect can spin a chimeric silk thread comprising an m-bFib H protein, preferably a chimeric Fib protein comprising an sFib protein, in the larval stage, particularly the late final instar stage.

A method of producing a chimeric silk thread of an m-bFib H protein and an sFib protein from the genome-modified silkworm of the present aspect may be in accordance with a conventional method of producing a silk thread from a silkworm. For example, a genome-modified silkworm may be allowed to spin a cocoon, from which a chimeric silk thread is prepared. A production method according to the present invention can mass-produce a chimeric silk thread of a bagworm silk thread and a silkworm silk thread using production facilities or the like for a silkworm silk thread, wherein the chimeric silk thread has the physical properties of a bagworm silk thread.

The method of producing a chimeric silk thread comprises a rearing process, a cocoon spinning process, a cocoon collecting process, and a reeling process as essential processes.

### (1) Rearing Process

The "rearing process" refers to a process of rearing the genome-modified silkworm of the present aspect. In the method of rearing a genome-modified silkworm, the silkworm may be reared in accordance with any silkworm rearing technique known in the art. See, for example, Takeo Takami, "Silkworm Eggs: An Overview," Japan Silkworm Egg Industry Association. As a diet, a natural leaf of a tree of a larval food plant species, such as trees belonging to the genus *Morus*, or an artificial diet, such as Silk Mate L4M or Silk Mate for the first to third instar parent silkworms (Nosan Corporation), may be used. An artificial diet is preferred because it can prevent disease development, provide a stable quality and quantity of diet, and also rear a silkworm under a sterile condition as necessary. A simple method of rearing the genome-modified silkworm will be described with reference to an example below.

Brushing-off is performed using eggs laid by an appropriate number of (for example, 4 to 10) female genome-modified silkworms of the same line. The larvae hatched from the eggs are transferred from a silkworm-moth-oviposition card onto a sheet of antidesiccation (paraffined) paper as a rearing bed in a container, and fed with an artificial diet such as the Silk Mate diet, which is placed on the sheet of antidesiccation paper. In principle, the diet is provided to the larvae once in each of their first and second instars and once to three times in their third instar. If a relatively large amount of stale food is left, the leftovers should be removed and prevented from going bad. For rearing grown silkworms in their fourth and fifth instars, the larvae are transferred into bigger containers, being adjusted to an appropriate number per container. A lid made from antidesiccation paper, acrylic resin, or mesh net may be placed on the container depending on the humidity and conditions inside the container. The rearing temperature is kept from 25 to 28°C throughout all the instars.

### (2) Cocoon Spinning Process

The "cocoon spinning process" refers to a process of allowing the genome-modified silkworm of the present aspect to spin a cocoon. The term "cocoon spinning" refers to the formation of a cocoon for pupation by a silkworm in its final (fifth) instar.

This process may be performed basically according to any cocoon spinning technique known in the art of silkworm rearing. This process can be accomplished by collecting matured silkworm larvae, for example, on the sixth to eighth days in their final instar and mounting those larvae on a mountage. The term "mounting" refers to transferring silkworms onto a mountage. The cocoon spinning may be performed at a temperature of 25 to 28°C. Then, the genome-modified silkworms form cocoons in the mountage.

### (3) Cocoon Collecting Process

The term "cocoon collecting process" refers to a process in which cocoons are separated off from a mountage and collected after the cocoon spinning process. This process also comprises removing the floss surrounding the cocoons. Cocoons may be collected 6 to 8 days after mounting larvae on a mountage. Cocoons can be collected manually, and are conveniently collected using a machine dedicated to collecting cocoons. Other than a floss removing machine which removes only cocoon floss, a fully automatic cocoon-collecting and floss-removing machine is available, wherein the automatic machine performs the processes from separating off cocoons from a mountage to removing floss from the cocoons.

### (4) Reeling Process

The term "reeling process" refers to a process of reeling a chimeric silk thread from the cocoons collected in the cocoon collecting process. The term "reeling" refers to producing a raw silk thread from cocoons. The cocoons collected are immersed in hot water at a temperature of approximately 95°C for "cocoon cooking" to unwind the cocoon filament, and "end groping" is performed in which the cocoon filament in entangled form is withdrawn from the surface of the cocoon using a groping brush. Then, "end picking" is performed to pick up one correct filament end from the cocoon groped, and the filament is reeled. These processes can be performed manually, but are preferably performed using a machine dedicated to reeling a thread, i.e., an automatic reeling machine. Through the above-described processes, a chimeric silk thread can be produced as a raw silk thread.

### 4. Chimeric Silk Thread

### 4-1. Overview

A fourth aspect of the present invention is a chimeric silk thread. The chimeric silk thread of the present aspect comprises a chimeric Fib protein in which an sFib H protein or an sFib L protein is linked with a bFib H protein. The chimeric silk thread of the present aspect can be obtained as a silk thread spun by the genome-modified silkworm according to the third aspect.

### 4-2. Constitution

A chimeric silk thread according to the present invention is constituted by a Fib H protein, a Fib L protein, and a p25 protein in the same manner as a usual silk thread, particularly a silkworm silk thread. After being spun, the chimeric silk thread before degumming further comprises a sericin protein, but this protein is removed by a degumming treatment.

A chimeric silk thread according to the present invention is characterized by being constituted by a chimeric Fib protein in which the m-bFib H protein is linked with the Fib H protein and/or the Fib L protein at an arbitrary position.

### Examples

### <Example 1: Preparation of Donor Vector>

### (Purpose)

The purpose is to prepare a donor vector that is to be comprised in a kit for producing a genome-edited silkworm according to the present invention.

### (Method)

A gene (SEQ ID NO: 28) encoding the bagworm Fib H protein (SEQ ID NO: 27) having 12 repeat units was prepared on the basis of the information on the sequence encoding a modified-bagworm Fib H protein consisting of the amino acid sequence of SEQ ID NO: 26 in Figure 2. Additionally, in a genome-editing donor vector (pBac[3XP3-DsRed2afm]E1LLL-EGFP: obtained from the National Agriculture and Food Research Organization), the sequence from the sFib L promoter sequence to the EGFP gene was replaced with the TALEN target sequence of SEQ ID NO: 29 (TAATGCTCAAAGATATATGCCAGCCAGGTGCACAAGCATTCACGTCTAAATACG AA) and the above-described bagworm Fib H gene located at the 3' side thereof, and furthermore, the DsRed2 gene was replaced with the EGFP gene to construct the donor vector pDVL-MMHX4 shown in Figure 3.

### <Example 2: Production of Genome-modified Silkworm>

### (Purpose)

The purpose is to produce a genome-modified silkworm of interest in accordance with a method of producing a genome-modified silkworm of the second aspect of the present invention.

### (Method and Results)

The host silkworm used was of a w1-pnd line. The silkworm was reared at 29°C until its fourth instar and at 25°C until its fifth instar in the following photocycle: a light period of 12 hours and a dark period of 12 hours. As a diet, an artificial diet (Silk Mate for the first to third instar parent silkworms: Nosan Corporation) was fed.

For injection, eggs within 6 hours after oviposition were used. The injection was performed by a conventional method using a solution prepared to have a donor vector at 500 ng/µL and each TALEN mRNA at 25 ng/µL. The eggs after injection were left to stand at 25°C until hatching. The eggs hatched were reared into imagoes by the same method as above-described. The imagoes obtained were denominated GOs (an injection generation). The G0s underwent sib mating, and the remaining G0 individuals were crossed with the parental-line (w1-pnd) individuals used for the injection. The female individuals after the crossing were allowed to oviposit. The G1 eggs obtained (the first generation after the injection) were left to stand at 25°C, and then protected at 5°C two days later.

The G1 eggs were left to stand at 25°C (delivery) 11 days before the expected date of hatching, and allowed to start embryogenesis. The transformants were screened using the eggs 5 to 7 days old after the delivery. Eggs visually recognized as having green fluorescence were selected as transformation-positive eggs.

The larvae hatched from the transformation-positive eggs were reared, and cocoons were collected 7 days after the mounting. Pupae were taken out of the cocoon-shells, and hatched. When this was done, one leg from which the genome was to be extracted was collected from an imaginal silkworm hatched for verifying the genome sequence, and each individual was managed in such a manner that the moths corresponded with the respective cocoon-shells. The imaginal silkworms were refrigerated in an unmated state until the PCR verification of the knock-in and the SDS-PAGE analysis of the cocoon-shell protein were completed as below-described.

To distinguish the individuals that underwent successful knock-in, PCR for amplification only in knock-in individuals was performed, using the primers that specifically bind to the silkworm Fib gene and the donor vector respectively at the 5'-side and 3'-side of the donor vector inserted into the genome. One leg was collected from the legs collected after the imagoes (*Bombyx mori*) were hatched. The genomic DNA was extracted using DNeasy Blood & Tissue Kit (Qiagen N.V.) in accordance with the attached protocol. The genomic DNA extracted was used as a template while PCR was performed under usual conditions, using KOD FX Neo (TOYOBO Co., Ltd.). The primers used were as follows.
(Insertion at 5' side)
   BmFib-LF: CAGACATATAAGAGCTACGA (SEQ ID NO: 30)
   MMFib-HR: TGATATTCGTCAGTGTCTGCT (SEQ ID NO: 31)
(Insertion at 3' side)
   SV40F: TGGTTTGTCCAAACTCATCA (SEQ ID NO: 32)
   BmFib-LR: CACAATTTGCATAAAATGTC (SEQ ID NO: 33)

The base sequence of the PCR product obtained was determined to verify whether the donor vector was correctly inserted at an accurate position in the silkworm Fib gene.

Part of the cocoon-shell of an individual was dissolved in 9 M lithium bromide, wherein the individual seemed to have the donor vector correctly inserted thereinto, according to the above-described base sequence. The resulting solution was subjected to SDS-PAGE of a silk thread protein, resulting in observing a protein having such a size as suggested a chimeric Fib protein of a silkworm and a bagworm. These individuals were crossed and systematized, and furthermore, the knock-in was verified by Western blotting. Using homo-individuals having two copies of donor vectors in the genome, hetero-individuals having one copy thereof, and wild-type individuals having no donor vector, the respective cocoon-shell proteins were separated in the same manner as in the above-described SDS-PAGE, and allowed to react with an anti-Fib-L antibody that binds to the N-terminal region of the silkworm Fib L protein. The above-described antibody, labeled with HRP (horseradish peroxidase), was used, and Amersham^{™} ECL^{™} Prime (Cytiva) was used for detection. With the cocoon-shell proteins of the homo-individuals and the hetero-individuals, a band (arrow) was observed at the position of the same chimeric Fib protein as in the above-described SDS-PAGE. On the other hand, with the cocoon-shell proteins of the hetero-individuals and the wild-type individuals, a band (arrowhead) was detected at the position of Fib L of the wild-type silkworm. From the above-described results, the silkworm line obtained in this Example was judged to be a genome-modified silkworm that produces a chimeric silk thread.

### <Example 3: Analysis of Physical Properties of Chimeric Silk Thread>

### (Purpose)

The purpose is to analyze the physical properties of a chimeric silk thread spun by the genome-modified silkworm produced in Example 2.

### (Method)

The cocoon of the genome-modified silkworm produced in Example 2 was cooked by a conventional method, and reeled using a multi-thread reeling machine (Harada Ltd.). Using a sizing reel, the raw silk thread obtained was measured off every time the thread was wound 30 times (33.75 m). The resulting threads were left to stand in a room at a temperature of 20°C and a humidity of 65*%* for 24 hours or more. Then, the fineness was determined from the weight measured. To analyze the toughness of the chimeric silk thread obtained, a sample that was among the raw silk thread products reeled off, and had the average fineness closest to the average fineness of the control section was used. As a control, a silkworm silk thread prepared under the same conditions was used. Using a TENSILON universal material testing instrument (RTG-1210, A&D Company, Limited), the breaking strength and the fracture elongation were measured 50 times per test, and the toughness was determined from the areas for the chimeric silk thread and the silkworm silk thread under the SS curve. The toughness refers to the amount of work (energy) required to fracture a thread, wherein the amount is given as the area under the stress-strain curve. In general, a higher toughness value means a higher degree of resistance to fracture.

### (Results)

As a result, it has been revealed that the toughness of the chimeric silk thread was enhanced 1.16 times, compared with the silkworm silk thread as a control. This demonstrates that a chimeric silk thread that is from a bagworm silk thread and a silkworm silk thread, and spun by a genome-modified silkworm produced according to the present invention is less prone to break than a usual silkworm silk thread.

All publications, patents, and patent applications cited herein should be incorporated herein by reference in their entirety.

Sequencing Listing

## Claims

1. A kit for producing a genome-modified silkworm that produces a chimeric fibroin protein comprising a silkworm-derived fibroin protein and a fibroin protein derived from one or a plurality of species of other silk-spinning insects,
the production kit comprising a Left-TALEN expression vector, a Right-TALEN expression vector, and a donor vector;
the Left-TALEN expression vector comprising a Left-TALEN coding region,
wherein the Left-TALEN coding region encodes, in the fibroin gene of the silkworm, a Left-TALE domain and a nuclease domain downstream of the Left-TALE domain,
wherein the Left-TALE domain recognizes and binds to the base sequence of the 5'-side TALE binding region located in the 5'-side vicinity of the insertion site of the fibroin gene of another silk-spinning insect;
the Right-TALEN expression vector comprising a Right-TALEN coding region,
wherein the Right-TALEN coding region encodes, in the fibroin gene of the silkworm, a Right-TALE domain and a nuclease domain downstream of the Right-TALE domain, wherein the Right-TALE domain recognizes and binds to the base sequence of the 3'-side TALE binding region located in the 3'-side vicinity of the insertion site of the fibroin gene of the another silk-spinning insect; and
the donor vector comprising a 5'-side TALE corresponding region, a 3'-side TALE corresponding region, a 5'-side homologous region, a 3'-side homologous region, and the fibroin gene derived from the another silk-spinning insect.

2. The production kit according to claim 1, wherein the donor vector comprises the 5'-side TALE corresponding region, the 3'-side homologous region, the 5'-side homologous region, and the 3'-side TALE corresponding region located in this order from the 5' side.

3. The production kit according to claim 1 or 2, wherein the fibroin protein derived from the another silk-spinning insect is a fibroin H chain protein.

4. The production kit according to any one of claims 1 to 3, wherein the another silk-spinning insect is a bagworm.

5. The production kit according to claim 4,
wherein the bagworm fibroin H chain protein is a modified fibroin H chain protein consisting of three or more same and/or different repeat units that are linked,
wherein the repeat unit consists of a full length of 120 to 178 amino acids, which comprise 30 or more G/A units each consisting of two amino acid residues, composed of a glycine residue and an alanine residue, and comprise an alanine cluster comprising 15 to 25 alanine residues at the N-terminal side of the repeat unit.

6. The production kit according to claim 5, wherein the alanine cluster consists of the amino acid sequence shown in SEQ ID NO: 3 or 4.

7. The production kit according to claim 5 or 6, wherein the repeat unit is any one or more selected from the amino acid sequences shown in SEQ ID NOs: 8 to 16.

8. The production kit according to any one of claims 4 to 7, wherein the gene encoding the bagworm fibroin H chain protein consists of any one of base sequence shown in SEQ ID NOs: 17 to 25.

9. A method of producing a genome-modified silkworm that produces a chimeric fibroin protein comprising a silkworm-derived fibroin protein and a fibroin protein derived from one or a plurality of species of other silk-spinning insects, the method comprising:
a nucleic acid introducing process of introducing a Left-TALEN mRNA or a Left-TALEN expression vector, a Right-TALEN mRNA or a Right-TALEN expression vector, and a donor vector into an egg of a silkworm;
a transformant selecting process of selecting, from the silkworm(s) after the nucleic acid introducing process, a transformant(s) comprising a fibroin gene derived from another silk-spinning insect; and
a genome-inserted individual selecting process of selecting, from the transformant(s), an individual wherein the fibroin gene of the another silk-spinning insect is inserted at a target position in the genome of the silkworm;
wherein the Left-TALEN mRNA comprises a Left-TALE region;
wherein the Left-TALE region encodes, in the fibroin gene of the silkworm, a Left-TAL domain and a nuclease domain downstream of the Left-TAL domain, wherein the Left-TAL domain recognizes and binds to the base sequence of the 5'-side TAL binding region at the insertion site of the fibroin gene of the another silk-spinning insect;
wherein the Left-TALEN expression vector comprises a promoter and the Left-TALE region located under the expression control by the promoter;
wherein the Right-TALEN mRNA comprises a Right-TALE region;
wherein the Right-TALE region encodes, in the fibroin gene of the silkworm, a Right-TAL domain and a nuclease domain downstream of the Right-TAL domain, wherein the Right-TAL domain recognizes and binds to the base sequence of the 3'-side TAL binding region at the insertion site of the fibroin gene of the another silk-spinning insect;
wherein the Right-TALEN expression vector comprises a promoter and the Right-TALE region located under the expression control by the promoter;
wherein the donor vector comprises a 5'-side TAL corresponding region, a 3'-side TAL corresponding region, a 5'-side homologous region, a 3'-side homologous region, and the fibroin gene of the another silk-spinning insect.

10. The production method according to claim 9, wherein the donor vector comprises the 5'-side TALE corresponding region, the 3'-side homologous region, the 5'-side homologous region, and the 3'-side TALE corresponding region located in this order from the 5' side on the basis of the direction of the sense strand of the fibroin gene of the another silk-spinning insect.

11. The production method according to claim 9 or 10, wherein the fibroin protein derived from the another silk-spinning insect is a fibroin H chain protein.

12. The production method according to any one of claims 9 to 11, wherein the another silk-spinning insect is a bagworm.

13. The production method according to claim 12,
wherein the bagworm fibroin H chain protein is a modified fibroin H chain protein consisting of three or more same and/or different repeat units that are linked,
wherein the repeat unit consists of a full length of 120 to 178 amino acids, which comprise 30 or more G/A units consisting of two amino acids composed of a glycine residue and an alanine residue, and comprise an alanine cluster comprising 15 to 25 alanine residues at the N-terminal side of the repeat unit.

14. The production method according to claim 13, wherein the alanine cluster consists of the amino acid sequence of SEQ ID NO: 3 or 4.

15. The production method according to claim 13 or 14, wherein the repeat unit is any one or more selected from the amino acid sequences of SEQ ID NOs: 8 to 16.

16. The production method according to any one of claims 12 to 15, wherein the gene encoding the bagworm fibroin H chain protein consists of any one base sequence of SEQ ID NOs: 17 to 25.

17. A genome-modified silkworm comprising a bagworm-derived fibroin H chain gene in the genome of the silkworm, wherein a fibroin H chain protein according to the gene is produced in a silk gland.

18. The genome-modified silkworm according to claim 17, wherein the bagworm-derived fibroin H chain protein is a chimeric fibroin protein comprising a silkworm-derived fibroin protein.

19. A chimeric silk thread comprising a chimeric fibroin protein wherein a modified bagworm fibroin H chain protein is linked at an arbitrary position with a silkworm fibroin H chain protein or L chain protein,
wherein the modified bagworm fibroin H chain protein comprises three or more same and/or different repeat units that are linked,
wherein the repeat unit consists of a full length of 120 to 178 amino acids, which comprise 30 or more G/A units consisting of two amino acids composed of a glycine residue and an alanine residue, and comprise an alanine cluster comprising 15 to 25 alanine residues at the N-terminal side of the repeat unit.

20. The chimeric silk thread according to claim 19, wherein the alanine cluster consists of the amino acid sequence of SEQ ID NO: 3 or 4.

21. The chimeric silk thread according to claim 19 or 20, wherein the repeat unit is any one or more selected from the amino acid sequences of SEQ ID NOs: 8 to 16.
